# EUROPEAN PATENT APPLICATION

(11) **EP 1 512 692 A2**
(43) Date of publication of application: **09.03.2005**
(21) Application number: 04077464.8
(22) Date of filing: 03.09.2004
(51) Int. Cl.: C07K 5/00, C07K 5/02, C07K 5/06, C07K 5/08, C07K 5/10, A61K 38/05, A61K 38/06, A61K 38/07, A61K 38/00, G01N 33/573, C12Q 1/48

(54) **Novel inhibitors of prenylated pyrophosphate consuming enzymes**

(30) Priority: 03.09.2003 US 653069
(71) Applicant: Universiteit Leiden, 2312 AV Leiden (NL); Nederlandse Organisatie voor Toegepast-Natuurwetenschappelijk Onderzoek TNO, 2628 VK Delft (NL)
(72) Inventor: Burm, Brigitta Elisa Anna, 3705 TD Zeist (NL); van der Elst, Hans, 2182 TV Hillegom (NL); Pieterman, Elsbet Jantine, 2312 KM Leiden (NL); Cohen, Louis Hartog, 3621 HR Breukelen (NL); Overhand, Mark, 2312 SZ Leiden (NL); van der Marel, Gijsbert Arie, 2318 MD Leiden (NL); van Boom, Jacobus Hubertus, 2343 CR Oegstgeest (NL); Voskuyl, Nicolette, 2152 LB Nieuw Vennep (NL); Eloualid, Farid, 3117 MK Schiedam (NL); Leroy, Ingrid Maria, 2807 GB Gouda (NL)
(74) Representative: van Westenbrugge, Andries

(57) **Abstract**

The invention pertains to new tripeptide and tetrapeptide analogs suitable as an inhibitor of a prenylpyrophosphate-consuming enzyme complying with formula A-B-B'-D, in which formula:
A is Ap-(CH₂)₀₋₃-Z¹-(CH₂)₀₋₃-Z²-(CHR¹)₀₋₁-CO-
B and B' are each independently a residue of a natural or unnatural amino acid, or B and B' together are a residue of a single non-natural amino acid having a cyclic unit;
D is -NH-CH(COX)-(CH₂)₀₋₂-R²
wherein Ap is an optionally substituted C₅-C₁₄ hydrocarbon group, R¹ is hydrogen or an optionally substituted C₁-C₄ alkyl group, R² is an optionally substituted aromatic or heterocyclic group or an aminoalkyl group, X is hydroxy or amino or their derivatives and Z¹ and Z² are direct bonds or spacer groups.

The invention further pertains to pharmaceutical compositions containing these peptide analogues and to the use of these analogues for treatment of conditions requiring inhibition of protein-prenyl transferase activity and for assaying protein-prenyl transferase activity.

## Description

The present invention relates to peptide analogues which inhibit prenylated pyrophosphate-consuming enzymes. Prenylated pyrophosphate consuming enzymes are understood to comprise protein:farnesyl transferases, protein:geranylgeranyl transferases and several other enzymes involved in the biosynthesis of terpenes, such as farnesyl pyrophosphate synthase, squalene synthase and geranylgeranyl pyrophosphate synthase.

### BACKGROUND

Prenylated pyrophosphates, i.e. *all-trans-*farnesyl pyrophosphate (FPP) and *all-trans*-geranylgeranyl pyrophosphate (GGPP), are substrates for a number of different enzymes. Protein:farnesyl transferase (PFT) is an enzyme which uses FPP to catalyse the farnesylation of cysteine residues near the C-terminus of certain proteins. The enzyme geranylgeranyl pyrophosphate synthase (GGPPS) uses FPP as a substrate in the production of geranylgeranyl pyrophosphate (GGPP), which is subsequently used in the synthesis of geranylgeranylated proteins mediated by the enzymes protein:geranyl-geranyl transferase 1 and 2 (PGGT-1 and 2).

Several of these prenylated (i.e. farnesylated and/or geranylgeranylated) proteins were identified as belonging to groups of related proteins: e.g. the nuclear lamins, low molecular weight GTP binding proteins (G-proteins), such as the *ras*-oncogene proteins and the γ-subunit of heterotrimeric G-proteins (Schäfer et al., *Science* **245** (1989) 379-385). Lamins and p21^{ras} proteins, 188 or 189 amino acid proteins which possess the consensus CaaX motif (C = cysteine, a = any amino acid having an aliphatic side chain and X = methionine, serine, glutamine or alanine) at the C-terminus, are farnesylated. Other small G-proteins, that have the consensus CaaL motif (L = leucine) such as Rho and several members of the *rab* proteins having C-terminal CC/CXC motifs, and also heterotrimeric G-protein γ-subunits are geranylgeranylated.

G-proteins are involved in the receptor-mediated transduction of signals (such as growth modulation signals) over the plasma membrane, and other prenylated proteins, not yet identified, may have a function in cell cycle progression. The prenylation of these proteins seems to play a role in their association with membranes and nuclear envelopes, where they are processed further and/or perform their function. This was shown for example by blocking the mevalonate synthesis by HMG-CoA reductase inhibitors, which prevented proteolytic processing of the lamin A precursor (Beck et al., *J. Cell. Biol.* **110** (1990) 1489-1499) or resulted, in other studies, in the accumulation of non-prenylated p21^{ras} precursor and the loss of transforming activity of oncogenic ras proteins. A review of the post-translational modification of proteins by isoprenoids in mammalian cells is given by Maltese W.A. in *FASEB J.* **4** 3319-3329 (1990). The latter observation triggered the search for specific inhibitors of the farnesylation of p21^{ras} in order to prevent its action in cells, where over-expression of this protein leads to tumour development, such as in colon carcinomas.

We have shown that FPP-analogues, which are *in vitro* inhibitors of PFT and/or PGGT-1, are able to inhibit the proliferation of human arterial smooth muscle cells in culture (Cohen et al. *Biochem. Pharm.* **57** (1999), 365-373) and are therefore potential inhibitors of the process of restenosis after percutaneous transluminal coronary angioplasty. The proliferation of smooth muscle cells plays a major role in the latter process.

Recently it was shown that inhibition of the cellular GGPP synthesis in specific bone cells (osteoclasts) is the possible mechanism of action of anti-osteoporosis drugs (Van Beek, E. *Biochem. Biophys. Res. Commun.* **255,** (1999), 491-494 and Van Beek, E. *J. Bone and Min. Res.* **14** (1999), 722-729). So, inhibitors of GGPPS and/or PGGT-1 and/or -2 may be active as anti-osteoporosis drugs as well.

Furthermore, G-proteins of the rab-family are involved in the regulation of intracellular protein traffic and secretion. In addition, prenylated proteins seem to play a role in the translational control of HMG-CoA reductase, the rate limiting enzyme of the isoprene and subsequent cholesterol biosynthesis. Squalene synthase (SS), the first pathway-specific enzyme in the biosynthesis of cholesterol, catalyses the reductive dimerisation of two molecules of FPP to produce squalene. Much attention has been directed to the development of inhibitors of SS with the aim to lower elevated blood plasma levels of cholesterol, one of the risk factors for cardiovascular diseases.

EP-A-540782 describes inhibitors for protein:farnesyl transferase based on prenyl pyrophosphate analogues. A review of new therapeutic methods based on protein:farnesyl transferase and inhibitors thereof are described by Leonard in *J*. *Med. Chem.* **40** (1997), 2971-2990. Furthermore, we recently (Overkleeft et al. *Tetrahedron Let.* **40** (1999) 4103-4107) described inhibitors of protein:prenyl transferases based on binding in the CaaX pocket of the enzyme concerned (see also EP-A-1028117). EP-A 1090909 discloses novel inhibitors of prenylated pyrophosphate consuming enzymes which are synthetic tetrapeptide analogues having a hydrophobic N-terminus and a C-terminus having an additional acid function. A typical example of these analogues is the compound phenylbutyryl-ε-aminocaproyl-aspartic acid (Ph-(CH₂)₃-CO-NH-(CH₂)₅CO--NH-CH(COOH)-CH(CH₃)-COOH.

### DESCRIPTION OF THE INVENTION

Novel analogues of prenyl pyrophosphate have been found according to the invention, which are inhibitors of prenylated pyrophosphate consuming enzymes (e.g. PFT, PGGT-1 and 2, GGPPS and SS) presumably by binding in the FPP pocket. The analogues are defined in the appending claims with reference to formula 1 (A-B -B'-D). The novel prenylated pyrophosphate-based inhibitors contain unnatural amino acid moieties which have the potential to be unsusceptible towards enzymatic degradation. These novel inhibitors are composed of amino acid derivatives which are ideally suited for the construction of a compound library *via* a combinatorial approach. Furthermore their peptide-like structure allows for easy access to bisubstrate analogues, in which the new FPP analogues are connected to CaaX box analogues, as defined in the appending claims.

The invention relates to inhibitors of prenyl pyrophosphate-consuming enzymes having a tripeptide or tetrapeptide structure A-B-B'-D, wherein
A is Ap-(CH₂)₀₋₃-Z¹-(CH₂)₀₋₃-Z²-(CHR¹)₀₋₁-CO-
B and B' are each independently a residue of a natural or unnatural amino acid, or
B and B' together are a residue of a single non-natural amino acid having a cyclic unit and/or having at least 6 chain atoms;
D is -NH-CH(COX)-(CH₂)₀₋₂-R².

In the formula A-B-B'-D, Ap is a hydrocarbon group containing 5-14 carbon atoms, optionally substituted with C₁₋₄ alkyl, C₁₋₄ alkoxy, halogen, methylenedioxy, nitro, aryl or aryloxy. The terminal X is OH, OM (M being an alkali metal), OR³, NH₂ or NHR³. The spacer Z¹ is -CH₂-CHR⁴-, -CH=CR⁴- or a direct bond, and the spacer Z² is-CO-NH-, -O-CO- or -O- or a direct bond. R¹ is hydrogen, or C₁₋₄ alkyl optionally substituted with amino, aryl or heteroaryl; R² is an aromatic or heterocyclic group, optionally substituted with C₁₋₄ alkyl, C₁₋₄ alkoxy, hydroxy, halogen or nitro, or a C₂₋₄ alkyl group substituted with amino, R³ is C₁₋₄ alkyl optionally substituted with hydroxyl or carboxyl; and R⁴ is H or C₁₋₄ alkyl.

The peptide analogues may also have the form of salts, such as metal and ammonium salts of the carboxylic acid groups and acid addition salts (from inorganic acids or organic acids) of the amino groups, as well as esters, such as alkyl, hydroxyalkyl and aryl esters.

Important features of the peptide-like FPP-analogues of the invention are the presence of a relatively hydrophobic region (part A), a spacer moiety comprising one or two (un)natural amino acids (parts B and B'). In particular, the C-terminal amino acid is an amino acid containing an aromatic, or heterocyclic or aminoalkyl group.

The N-terminal group A contains at least six carbon atoms (including further groups such as a carbonyl or oxymethylene group) up to about 24 carbon atoms, preferably between 8 and 18 carbon atoms, and may comprise alkyl, alkenyl, cycloalkyl, cycloalkenyl or aryl groups which may also contain heteroatoms. These groups may contain substituents, which are preferably non-polar or non-ionogenic, such as C₁-C₄ alkyl, C₁-C₄ alkoxy, halogen, haloalkyl, nitro and the like. Examples of non-aromatic moieties are hexyl, isooctyl, decyl, dodecyl, hexadecyl, cyclopentenyl, cyclohexyl, methylcyclohexyl, tetrahydropyranyl, and extensions thereof such as cyclohexylmethylcarbonyl, cyclohexylethyl, lauroyl, and the like. Aryl and especially aralkyl groups are preferred. Aromatic moieties include phenyl, tolyl, methoxyphenyl, isopropoxyphenyl, methylenedioxyphenyl, nitrophenyl, pentafluorophenyl, tetrahydronaphthyl, pyridyl, pyrimidyl, furyl, imidazolyl, thiazolyl, naphthyl, phenoxyphenyl, indolyl, anthryl, and the corresponding benzoyl, phenylethyl, cinnamyl and longer aralk(en)yl derivatives. Examples of part A further include phenyl-(C₁-C₆)-alkanoyl, benzoylaminoacetyl, furoylaminoacetyl, β-(benzoylamino)acryloyl, *N*-(benzyloxycarbonyl)-histidinoyl, and naphthyl-acetyl.

The total length of the spacer (parts B and B') is preferably 6-12 atoms for PFT inhibitors and longer, up to 18 atoms, for PGGT-1 or -2 inhibitors, the spacer may optionally contain multiple amide/amine linkages as well as other heteroatoms (e.g. S, O, etc.) and may be flexible or conformationally restricted. Preferably, B and/or B' are residues of unnatural amino acids, such as *N*-alkyl amino acids (e.g. *N*-methylglycine) aliphatic β-, γ-, δ-, and other ω-amino acids, the corresponding α,ω-diamino acids and other substituted ω-amino acids, such as β-asparagine, γ-glutamine or their *N*'-substituted derivatives, or amino acids containing a cyclohexylene or phenylene or heterocyclic moiety between the amino group and the carboxyl group. A single non-natural amino acid residue may be sufficient as a spacer B-B', if this amino acid contains a bulky and/or cyclic group, such as thiazol(idin)yl, piperidyl, pyridyl, phenyl, amino group and the carboxyl group. A single non-natural amino acid residue may be sufficient as a spacer B-B', if this amino acid contains a bulky and/or cyclic group, such as thiazol(idin)yl, piperidyl, pyridyl, phenyl, and especially (tetrahydro)naphthyl, (dihydro)indolyl or (tetrahydro)(iso)quinolyl, and/or if this amino acid contains at least 6 chain atoms. Natural amino acids comprise the 20 α-amino acids occurring in mammalian biochemistry, although cysteine and the acidic amino acids glutamic and aspartic acid are even less preferred in the spacer than the other natural amino acids. General examples of suitable amino acids B and B' are given in and below table 1.

According to a particular aspect of the invention, the carboxy-terminal residue (D) contains an aromatic or heterocyclic group, or a C₂₋₅ alkyl group substituted with amino. The aromatic group may e.g. phenyl, pyridyl, pyrimidyl, imidazolyl, thiazolyl, oxazolyl, and their benzo analogues such as naphthyl, quinolyl, isoquinolyl and indolyl. The aromatic group may be substituted with common groups such as C₁₋₄ alkyl, C₁₋₄ alkoxy, hydroxy, halogen, amino or nitro and partially hydrogenated such as in tetrahydronaphthyl. Other heterocyclic groups than the aromatic heterocycles mentioned above include piperidino, piperazino, and other hydrogenated analogues from the above heterocycles. Examples include phenyl, imidazolyl, indolyl etc, leading to natural (e.g. phenylalanine, tryptophan) or unnatural (e.g. phenylglycine, piperidinoalanine) amino acids. The amino-substituted alkyl groups may result in the amino acid being e.g. lysine, other α,ω-diamino acids or arginine.

Preferred peptide analogues according to the invention are analogues that are expected to be specific for the inhibition of farnesylation or geranylgeranylation processes as a function of their total length. It is anticipated that specific SS and GGPPS inhibitors will be obtained from the screening of a compound library due to the different mechanisms in which these enzymes convert FPP.

The peptide analogues according to the invention can be prepared in a manner which is known per se, starting from the individual (amino) acid building blocks A, B, B' and D in (amino)-protected form. Their synthesis can be analogous to the synthesis of the analogues described in EP-A 1090909. For example, they may be prepared by reacting the suitably protected building block for B having the formula:

Fmoc-NH-[Y]-COOH

with the protected building block for B' having the formula:

Fmoc-NH-[Y']-CO-tBu

to produce the dipeptide with formula

Fmoc-NH-[Y]-CO-NH-[Y']-CO-tBu

and coupling this unit to the terminal units :

Ap-(CH₂)₀₋₃-Z¹-(CH₂)₀₋₃-Z²(CHR¹)₀₋₁COOH

and

NH₂-CH(COO-tBu)-(CH₂)₀₋₂-R²

to produce the desired analogue with the formula

Ap(CH₂)₀₋₃Z¹-(CH₂)₀₋₃Z²(CHR¹)₀₋₁CO-NH-[Y]-CO-NH-[Y']-CO-NH-CH(COOH)-R²

wherein [Y] and [Y'] are the chain units of B and B', Fmoc is fluorenylmethoxycarbonyl or another protecting group, and using the appropriate deprotection steps.

It was found that the peptide analogues according to the invention are capable of inhibiting the protein-farnesylation and/or geranylgeranylation process. Because of their ease of synthesis various inhibitors can be easily generated through combinatorial chemistry. The bioavailability, stability against enzymatic degradation and inhibitory activity of these novel inhibitors will be better than for previously reported compounds.

The peptide-like FPP-analogues described above are useful as an active substance in a pharmaceutical composition intended to interfere with protein prenylation or cholesterol biosynthesis. As such, they are useful as inhibitors in processes such as oncogenesis and other unwanted cell proliferation, e.g. in restenosis or atherosclerosis, and furthermore as suppressants of aberrant high signal transduction. Also, they can be used as anti-osteoporosis drugs and drugs to lower elevated plasma levels of cholesterol.

The pharmaceutical compositions containing the analogues according to the invention may be formulated in a usual way, e.g. by associating the inhibitors with a suitable solid or liquid carrier and optional adjuvants or other active components. The composition may be suitable for oral administration (capsule, pill, tablet, gel, powder, sachet, syrup, solution, dispersion etc.) or may be an injectable solution or another administration form. The composition may be administered to mammalians including man, in a dose which depends on the particular purpose of the administration and other conditions well known to the skilled person. A suitable dose is e.g. from 1 to 500 mg/kg body weight, especially from 10 to 200 mg/kg body weight. A dose can be administered in a single dosage or in several daily dosages.

The invention thus also pertains to prophylactic and therapeutic methods for the treatment of subjects suffering from or threatened by diseased states as described above, comprising administering to a subject on need of said treatment an effective dose of the analogue or the pharmaceutical composition containing it.

The analogues of the invention can also be used in diagnostics procedures involving of assaying protein:prenyl-transferase activity in a biological sample or any other prenyl pyrophosphate consuming enzyme, by contacting the sample in which such activity is be determined with the peptide analogue and further reagents, enzyme substrates, labels (dyes, radioactive or fluorescent labels, antibodies etc.), diluents and the like as necessary for detecting the interaction of the peptide analogue with the enzyme under investigation.

### EXAMPLES

### Example 1: Synthesis of protein farnesyl and/or geranylgeranyl transferase inhibitors: Dbo-Lys-Eaca-Nphas-Phe

The synthesis of tetrapeptide analogue [bis(benzyloxycarbonyl)lysyl]-[ε-aminocaproyl]-[β-(nitrophenylaminocarbonyl)-β-alanyl]-[phenylalanine] started from protected phenylalanine, using a standard solid phase peptide synthesis protocol (Atherthon et al. *Solid Phase Synthesis: A Practical Approach,* IRL Press: Oxford, 1989), including reaction with 20% piperidine (pip) in dimethylformamide (DMF) and reaction with benzotriazole-1-yl-oxy-tris(dimethylamino)phosphonium hexafluorophosphate (BOP), 1-hydroxybenzotriazole (HOBt), diisopropylethylamine (DiPEA) in N-methylpyrrolidone (NMP) or with acetic anhydride (Ac₂O), HOBt/DiPEA/NMP. Thus the protected phenylalanine was coupled to Wang resin and then reacted with N-protected N'-nitrophenyl β-asparagine to produce the corresponding resin-anchored dipeptide analogue. This was reacted with activated N-protected ε-aminocaproic acid to produce the corresponding resin-anchored tripeptide analogue. This was then reacted with activated N,N'-bis(benzyloxycarbonyl)lysine to give the resin-anchored tetrapeptide, which was finally cleaved from the resin by hydrolysis using with trifluoroacetic acid (TFA)/triisopropylsilane (iPr₃SiH), yielding the title tetrapeptide analogue. This was purified using preparative HPLC and characterised spectroscopically.
Other compounds including those summarised in table 1 were synthesised and purified following the same scheme.

### Example 2: Assays of protein:farnesyl transferase and protein:geranylgeranyl transferase 1

PFT activity was determined using a C-terminal peptide of pre-p21^{*N-ras*} coupled to sepharose beads as substrate (pepAsep) as described previously (Cohen et al. *Biochem.* *Pharmacol.* **57** (1999), 365-373). The experimental conditions (25 µL reaction mixture) were as follows: 80 pmol/25 µL sepharose-coupled peptide, 0.7 µM of [³H]-FPP (American Radiolabeled Chemicals Inc.; specific radioactivity 15 Ci/mmol) and 13 µL of rat brain enzyme preparation. Incubation was performed at 37°C for 30 min. For the determination of IC₅₀ values of the FPP analogues, the assay was performed three times in the presence of different concentrations of particular compound in duplicate.
Determination of PGGT-1 activity was performed in a similar manner as described for PFT activity and has also been described previously (Cohen et al. *Biochem. Pharmacol.* **57** (1999), 365-373). The same C-terminal peptide was used, except that the C-terminal methionine was replaced by leucine (pepCsep). The experimental conditions were as follows: 25 µL incubation mixture contained 2.5 µL (1 nmol) pepCsep, 1 µM [³H]-GGPP (American Radiolabeled Chemicals Inc.; specific radioactivity 15 Ci/mmol), 3 µL of bovine brain enzyme preparation, 50 µM ZnCl₂, 0.5 mM MgCl₂, 1 mM dithiothreitol, 0.004% Triton X-100, 50 mM Tris-HCl (pH 7.4). Incubation was performed at 37°C for 40 min. with continuous shaking. For the determination of IC₅₀ values of the GGPP analogues, the assay was performed three times in the presence of at least five different concentrations of the particular compound in duplicate.

### Example 3

The relative inhibition activity of the compounds mentioned in table 1 on PGGT was assayed using the test described below in percentage PGGT activity of the control (without the analogue). The results are summarised in table 1.

***Assay of protein:geranylgeranyl transferase-1*** *(as described in: Cohen L.H., et al. Biochem. Pharmacol. 57, 1999, 365-373).* Determination of PGGT-1 activity was performed by using a sepharose coupled peptide as substrate. The amino acid sequence of the peptide was the same as described for the PFT assay (pepAsep; Cohen L.H., et al. Biochem. Pharmacol. 49, 1995, 839-845) except that the C-terminal methionine has been replaced by leucine, introducing the CaaL-box, which is the consensus sequence for geranylgeranylation by PGGT-1. This substrate has been designated as pepCsep. PepBsep, the non-isoprenylatable sepharose-coupled peptide (see PFT assay), was used as a control to measure non-specific association of radiolabeled GGPP. A crude PGGT-1 preparation was isolated from bovine brain according to Yokoyama et al. (Proc. Natl. Acad, Sci. USA 88, 1991, 5302-5306).

The radioactivity bound to the sepharose was strongly dependent on the presence of the cysteine residue in pepCsep and the reaction was linear up to 40 min and up to 5 µL of the enzyme preparation. On the basis of these data the conditions for the determination of PGGT-1 activity were as follows: The incubation mixture (25 µL) contained 2.5 µL of pepBsep or pepCsep (1 nmol of peptides), 3 µL of bovine brain enzyme, 1 µM of [3H]-GGPP (spec. radioactivity 15 Ci/mmol, American Radiolabeled Chemicals, USA), 50 µM ZnCl₂, 0.5 mM MgCl₂, 1 mM DTT, 0.004 % Triton X-100, 50 mM Tris-HCl (pH 7.4). For the determination of the inhibitory potencies of the various compounds, 2 to 5 different concentrations of these compounds were added to the mixture. The incubation was performed at 37° C for 40 min with continuous shaking. The reaction was terminated by addition of 1 ml of 2% (w/v) of SDS, the beads were spun down and washed successively 3 times with 2 % (w/v) SDS under shaking for 45 min at 50° C. For the calculation of PGGT-1 activity the 3H-counts bound to pepBsep were subtracted from the counts bound to pepCsep. For the determination of the IC₅₀ values of the FPP analogues, the assay was repeated at least three times in the presence of the various concentrations of the compounds and the concentration at 50% inhibition was determined using a mathematical function fitting to the concentration/inhibition curve.

**Table I:**

| *Peptide analogues and their geranyl-geranyl transferase inhibiting properties; Relative activity in % activity with respect to control* | | | | | | |
|---|---|---|---|---|---|---|
| *Code* | *A* | *B* | *B'* | *D* | *relative activity at 10 µM* | *relative activity at 100 µM* |
| 219 | Phcap | Amoc | Aibu | Lys | 60 ±1 | 37 ± 0 |
| 319 | Phbu | Eaca | - | His | 82 ± 2 | 35 ± 2 |
| 343 | Boc-His | Amoc | Eaca | His | 53 ± 9 | 29 ± 7 |
| 241 | Palm | γDabu | Lys | Trp | 73 ±8 | 61 ± 57 |
| 272 | Nben | Amoc | αDava | Trp | 20 ± 1 | 16 ± 2 |
| 273 | Palm | γDabu | Thisq | Trp | 81 ± 7 | 47 ± 10 |
| 274 | Palm | Bala | Lys | Pgly | 19 ± 2 | 5 ± 2 |
| 310 | Palm | Pazac | Lys | Pgly | 75 ±6 | 36 ± 1 |
| 370 | Palm | αDabu | Lys | Pgly | 3 ± 2 | 32 ± 1 |
| 280 | Boc-His | 3Pip | Aibu | Nphe | 63 ±3 | 25 ±4 |
| 358 | Boc-His | 3Pip | Bala | Nphe | 74 ±3 | 27 ± 4 |
| 250 | Mphac | Ambz | - | Tyr | 87 ± 1 | 76 ± 4 |
| 293 | Mphac | Thiaz | Bala | Pipa | 72 ±6 | 36 ± 5 |

### Abbreviations:

| *Units A* | |
|---|---|
| Boc | Benzyloxycarbonyl C₆H₅CH₂-O-CO- |
| Dbo-Lys | *N*^{α},*N*^{ε}-Bis(benzyloxycarbonyl)-lysyl Boc-NH-(CH₂)₄-CH(NH-Boc)-CO- |
| Mphac | p-Methoxyphenylacetyl *p*-CH₃C₆H₄CH₂-CO- |
| Nben | p-Nitrobenzoyl *p*-NO₂-C₆H₄-CO- |
| Palm | Palmitoyl CH₃-(CH₂)₁₀-CO- |
| Phbu | Phenylbutyryl C₆H₅(CH₂)₃-CO- |
| Phcap | Phenylcaproyl C₆H₅(CH₂)₅-CO- |

| *Units B, B'* | |
|---|---|
| Aibu | α-Aminyleneisobutyryl -NH-C(CH₃)₂-CO- |
| Ambz | 3-Aminylenemethyl-benzoyl -NH-CH₂-*m*C₆H₄-CO- |
| Amch | 4-Aminylenemethyl-cyclohexanecarbonyl -NH-CH₂-C₆H₁₀-CO- |
| Amoc | 8-Aminyleneoctanoyl -NH-(CH₂)₇-CO- |
| Aval | δ-Aminylenevaleroyl -NH-(CH₂)₄-CO- |
| Bala | β-Alanyl -NH-CH₂CH₂-CO- |
| Gaba | γ-Aminylenebutryryl -NH-(CH₂)₃-CO- |
| αDabu | α-Aminylene-γ-aminobutyryl -NH-CH((CH₂)₂NH₂)-CO- |
| γDabu | γ-Aminylene-α-aminobutyryl -NH-(CH₂)₂-CH(NH₂)-CO- |
| αDava | α-Aminylene-δ-aminovaleroyl -NH-CH((CH₂)₃NH₂)-CO- |
| δDava | δ-Aminylene-α-aminovaleroyl -NH-(CH₂)₃-CH(NH₂)-CO- |
| Eaca | ε-Aminylenecaproyl -NH-(CH₂)₅-CO- |
| εLys | ε-Aminylene-α-aminocaproyl -NH-(CH₂)₄-CH(NH₂)-CO- |
| Nphas | β-Aminylene-*N*'-(p-nitrophenyl)--succinoyl -NH-CH(CONHC₆H₄NO₂)CH₂CO- |
| Pazac | Piperazino-4-acetyl -N(C₂H₄)₂N-CH₂-CO- |
| 3Pip | Piperidino-3-carbonyl -N(CH₂)(C₃H₆)CH-CO- |
| 4Pip | Piperidino-4-carbonyl -N(C₂H₄)₂CH-CO- |
| Thiaz | Thiazolidine-3-yl-4-ylcarbonyl -N(CH₂SCH₂)CH-CO- |
| Thisq | Tetrahydroisoquinoline-2-yl-3-ylcarbonyl -(NC₉H₉)-CO- |

| *Units D* | |
|---|---|
| Nphe | p-Nitrophenylalanine -NH-CH(COOH)-CH₂-*p*C₆H₄-NO₂ |
| Pgly | Phenylglycine -NH-CH(COOH)-C₆H₅ |
| Pipa | Piperidinoalanine -NH-CH(COOH)-CH₂-NC₅H₁₀ |

## Claims

1. A peptide analogue suitable as an inhibitor of a prenylpyrophosphate-consuming enzyme complying with formula A-B-B'-D (1):
in which formula:
A is Ap-(CH₂)₀₋₃-Z¹-(CH₂)₀₋₃-Z²-(CHR¹)₀₋₁-CO-
B and B' are each independently a residue of a natural or unnatural amino acid, or
B and B' together are a residue of a single non-natural amino acid having a cyclic unit and/or having at least 6 chain atoms;
D is -NH-CH(COX)-(CH₂)₀₋₂-R²
in which:
Ap is a hydrocarbon group containing 5-14 carbon atoms, optionally substituted with C₁₋₄ alkyl, C₁₋₄ alkoxy, halogen, methylenedioxy, nitro, aryl or aryloxy;
X is OH, OM (M being an alkali metal), OR³, NH₂ or NHR³;
Z¹ is -CH₂-CHR⁴-, -CH=CR⁴- or a direct bond;
Z² is -CO-NH-, -O-CO- or -O- or a direct bond;
R¹ is hydrogen, or C₁₋₄ alkyl optionally substituted with amino, aryl or heteroaryl;
R² is an aromatic or heterocyclic group, optionally substituted with C₁₋₄ alkyl, C₁₋₄ alkoxy, hydroxy, halogen or nitro; or a C₂₋₄ alkyl group substituted with amino;
R³ is C₁₋₄ alkyl optionally substituted with hydroxyl or carboxyl,
R⁴ is H, or C₁₋₄ alkyl;
or a salt or ester thereof.

2. A peptide analogue according to claim 1, wherein at least one of B and B' is a residue of an unnatural amino acid.

3. A peptide analogue according to claim 1 or 2, wherein said unnatural amino acid is selected from ω-amino C₂₋₈ alkanoic acids and cyclic amino acids.

4. A peptide analogue according to any one of claims 1-3, wherein in D, R⁵ is selected from piperidino, piperazino, phenyl, nitrophenyl, pyridyl, pyrimidyl, imidazolyl, thiazolyl, naphthyl, quinolyl and indolyl.

5. A peptide analogue according to any one of claims 1-4, wherein D is phenylglycine, phenylalanine, nitrophenylalanine, piperidinoalanine, tryptophan, or lysine.

6. Use of a peptide analogue according to any one of claims 1-5 as a medicament.

7. Use of a peptide analogue according to any one of claims 1-5 for preparing a pharmaceutical composition for the treatment of conditions requiring inhibition of the activity of protein:prenyl-transferases or any other prenyl pyrophosphate consuming enzyme.

8. Use according to claim 7, wherein said conditions comprise osteoporosis, atherosclerosis, restenosis or cancer.

9. A pharmaceutical composition containing a peptide analogue according to any one of claims 1-5, together with a pharmaceutically acceptable carrier.

10. A method of assaying protein:prenyl-transferase activity in a biological sample or any other prenyl pyrophosphate consuming enzyme, comprising contacting said sample with a peptide analogue according to any one of claims 1-5.
